Europäisches Patentamt

European Patent Office

Office européen des brevets

(19)

(11)     EP 1 079 840 B1

(12)     **EUROPEAN PATENT SPECIFICATION**

(45) Date of publication and mention
of the grant of the patent:
**02.10.2002   Bulletin 2002/40**

(51) Int Cl.[7]: **A61K 31/70**, A61K 31/505,
A61K 31/52, A61K 31/17,
A61K 31/715

(21) Application number: **99924999.8**

(22) Date of filing: **18.05.1999**

(86) International application number:
**PCT/EP99/03399**

(87) International publication number:
**WO 99/059600 (25.11.1999 Gazette 1999/47)**

(54) **SYNERGISTIC COMPOSITION OF INULIN AND AN ANTI-CANCER DRUG FOR USE IN THE TREATMENT OF CANCER**

SYNERGISTISCHE ZUSAMMENSETZUNG ZUR BEHANDLUNG VON KREBS, WELCHE INULIN UND EIN ANTIKREBSMITTEL ENTHÄLT

COMPOSITION SYNERGIQUE CONTENANT DE L'INULINE ET UN MEDICAMENT ANTI-CANCER UTILISEE POUR TRAITER LE CANCER ANCER

(84) Designated Contracting States:
**AT BE CH DE DK ES FR GB IE IT LI NL PT SE**

(30) Priority: **18.05.1998   EP 98870113**

(43) Date of publication of application:
**07.03.2001   Bulletin 2001/10**

(73) Proprietor: **Tiense Suikerraffinaderij N.V.
(Raffinerie Tirlemontoise S.A.)
1150 Brussel (BE)**

(72) Inventors:
• **TAPER, Henryk
B-3012 Wilsele (BE)**
• **FRIPPIAT, Anne
B-1933 Sterrebeek (BE)**
• **VAN LOO, Jan
B-3040 St. Agatha Rode (BE)**

• **ROBERFROID, Marcel
B-1348 Louvain-La-Neuve (BE)**

(74) Representative: **Hermans, Johny
Tiense Suikerraffinaderij N.V.,
Patent Department,
Aandorenstraat 1
3300 Tienen (BE)**

(56) References cited:
**EP-A- 0 692 252**

• **LEIBOVICI J. ET AL: "Combined effect of levan and cytotoxic agents on the growth of experimental tumours in mice" BR. J. EXP. PATHOL., 1983, 64/3 (239-244), XP002083711 ENGLAND cited in the application**

**Description**

Field of the invention

This invention relates to a pharmaceutical composition comprising a combination of a non-digestible carbohydrate and an anti-cancer drug.

Background and prior art

For various reasons, cancer has become one of the major causes of death of humans mainly in highly developed and highly industrialised countries.

Several kinds of cancer have already been identified. Although not all mechanisms of the carcino-genesis and development of all kinds of cancer have been elucidated so far, cancer is a disease which is known to proceed in several steps, conventionally including the stages of initiation, promotion and progression. The initiation stage is characterised by a neoplastic modification of the genome in normal cells. In the promotion stage, the initiated cells with altered genome express their altered genome by a phenotypically detectable proliferation ( premalignant foci, nodules or benign neoplasms). In the stage of progression, the benign neoplastic cells are transformed into malignant tumor cells proliferating in an uncontrolled manner, locally invading adjacent normal body tissues and organs and finally spreading into distant body tissues and organs by the metastases. The invasion of normal body structures usually results in their malfunctioning and destruction, which eventually results in the death of the affected human.

Cancer is also often seen in mammals in which the disease generally occurs through a mechanism similar to the one in humans.

On the one hand, various methods for the treatment of cancer have already been developed including treatment by surgery, by irradiation (X-rays, gamma rays, isotopes), by chemotherapy or, frequently, by combinations thereof.

The method of treatment of humans is commonly chosen taking into account *inter alia* the age and physical condition of the affected human and the kind, the. location and the stage of development of the cancer. In a few cases, the treatment will be effective and able to completely cure the affected person. In most cases, however, the treatment can only delay the carcinogenesis or more or less inhibit the development of the cancer disease and, in spite of bringing some temporary relief to the patient, the final outcome is unfortunately still fatal.

The treatment methods are continuously improving as a result of major research efforts. Invasive techniques, including surgery and irradiation, become more and more efficient as a result of technological developments, but require always more highly sophisticated equipment, making the techniques very costly and less suitable for large scale application. In the search to overcome the drawbacks of invasive treatments as well as for various other reasons, chemotherapy, in combination or not with other treatment methods, has become a major way of attack against cancer during the last two decades. A considerable advantage of anti-cancer drugs resides in the fact that they are easy to administer and current developments enable, in a steadily improving manner, target delivery of the drugs.

The anti-cancer drugs which have been developed are generally classified in one of the following groups:

1. alkylating agents, including e.g. cyclophosphamide (Endoxan®);
2. anti-metabolic drugs, including e.g. 5-fluorouracil and methotrexate;
3. anti-mitotic antibiotics, including e.g. doxorubicine and adriamycin;
4. alcaloidal anti-tumor agents, e.g. vincristine sulphate (Oncovin®);
5. hormones and antihormones,
6. interferons, e.g. alfa-2 b interferon; and
6. various anti-tumor agents,

as disclosed for example in the Répertoire commenté des médicaments, Ed. Centre Belge d'information pharmacothérapeutique, (1987), p. 341-345.

However, most of the anti-cancer drugs have serious disadvantages and drawbacks too. They may present a high degree of toxicity for cells of normal body structures causing, for example, liver and kidney damages. They may also cause an increased sensitivity to opportunistic infections. Often they also provoke various types of discomfort for the treated person, such as, local necrosis of the body structure in which the drug is parenterally administered, and, when the drug is administered orally or via tube feeding, nausea, vomiting, irritation of the mucoses of the digestive tract and diarrhoea. In addition to discomfort commonly caused by anti-cancer drugs, anti-metabolic anti-cancer drugs are known to be fairly toxic and to provoke additional discomfort, including megaloblastose, and lesions to the liver or the digestive tract such as stomatitis and buccal and gastro-intestinal ulcers.

The disadvantages and drawbacks may considerably limit the use of available anti-cancer drugs. Indeed, often a curative effective dose of the drug can not be given to a patient due to the too high toxicity of the drug to normal cells or to the too high degree of discomfort caused to the patient by the drug.

On the other hand, beneficially inhibitory effects of certain dietary components on the incidence and growth of cancer have been disclosed *inter alia* by Williams and Dickerson (1990); Roberfroid (1991) and Milner (1994 ). Said components, termed functional food components (Roberfroid, 1995), may have almost no nutritional and/or caloric value but interact with physiological functions in the body in a manner to improve them or to restore them more or less in case of a disfunction.

Amongst the many dietary components, non-digestible carbohydrates, commonly termed dietary fibres, have been found to present preventive effects on carcinogenesis (Nossal, 1993; Perdigon et al.,1993) and possibly inhibitory effects on the promotion of carcinogenesis (Wattenberg, 1992).

Moreover, the relation between the intake of dietary fibres and the reduction of the risk of colon cancer has been disclosed in several publications, e.g. Potter et al., (1996); Howe et al., (1992) and Reddy et al., (1992).

Dietary fibres are commonly defined as components of plant cells which are resistant to hydrolysis by the alimentary enzymes of humans and non ruminating mammals. Dietary fibres comprise cellulose, hemi-cellulose, pectin, gums, waxes, lignin and certain other non-digestible carbohydrates.

Fructans are carbohydrates which are commonly classified in levans and inulins.

Levans are D-fructans generally consisting of water soluble chains of fructose units which are mostly connected to each other by $\beta$(2-6) fructosyl-fructose linkages. Levans originate from the activity of certain bacteria and can be produced by known fermentation techniques as well as by enzymatic synthesis. Levans usually occur as a polydisperse mixture of polyfructose chains. These chains may be linear, but in general they are branched.

As $\beta$(2-6) fructosyl-fructose linkages are not hydrolysed by alimentary enzymes in the upper part of the digestive tract of humans and non ruminating mammals, levans pass almost unaltered into the colon where they are fermented by intestinal bacteria.

Inulins are D-fructans consisting of water soluble chains of fructose units too, but they are composed of chains in which the fructose units are connected to each other mostly or exclusively by $\beta$(2-1) fructosyl-fructose linkages. Besides, inulin-type polyfructose chains often end in a glucose unit. Inulin mostly occurs as a polydisperse mixture of linear polyfructose molecules, as for example inulin from chicory, but inulin can also occur as a polydisperse mixture of branched polyfructose molecules, as for example inulin from dahlia and inulin from agave.

Inulins can be represented by the general formulae $GF_n$ and $F_n$ wherein G represents a glucosyl unit, F represents a fructosyl unit, and n represents the number of fructosyl units linked to each other in the carbohydrate chain. The number of saccharide units (fructose and glucose units) in one inulin molecule, *i.e.* the values n+1 in formula GFn and n in formula Fn, are referred to as the degree of polymerisation, represented by (DP). Often the parameter average degree of polymerisation of the inulin, represented by $(\overline{DP})$, is used too, which is the value corresponding to the total number of saccharide units divided by the total number of inulin molecules present in a given composition, without taking into account possibly present mono- and disaccharides (De Leenheer, 1996).

Inulins are synthesised by many plant species, in parts of which they may be stored as reserve carbohydrates, but inulins can also originate from bacterial activity and can be enzymatically synthesised as well. Inulin from plant origin is a polydisperse composition of polyfructose chains with a degree of polymerisation (DP) ranging from 3 to about 100, whereas inulin from bacterial origin usually has a higher (DP). The inulin profile, i.e. the distribution profile of the inulin chains according to their (DP), which to a certain extent is indicated by the average degree of polymerisation $(\overline{DP})$, depends from various factors, including the plant species, the time in the plant life cycle when the plant part is harvested, and the way the plant part is processed into inulin.

In roots of chicory, in tubers of dahlia and Jerusalem artichoke, and in the pinia of agave, inulin can occur at concentrations of about 10% to about 20 % on fresh weight. Inulin can be extracted easily from these plant parts, for example by extraction of the shredded plant parts with warm water. Then the crude inulin is purified and optionally fractionated to remove undesired fractions of carbohydrates. Finally, the inulin is isolated in particulate form, usually by spray drying or by directed crystallisation. These different process steps are well known in the art. At industrial scale, inulin is mainly obtained from roots of chicory.

Inulin from chicory is commercially available in various grades, for example as RAFTILINE® from ORAFTI, (Tienen, Belgium). Typical RAFTILINE® grades are, for example , ST (which has a $(\overline{DP})$ of about 10 and contains in total about 8 % by weight glucose, fructose and sucrose), LS (which also has a $(\overline{DP})$ of about 10 but which contains in total less than 1 % by weight glucose, fructose and sucrose), and HP (which has a $(\overline{DP}) \geq 23$, commonly a $(\overline{DP})$ of about 25 to about 30, and is essentially free of glucose, fructose and sucrose).

Inulin-type molecules of general formulae $GF_n$ and $F_n$ having a lower degree of polymerisation (DP), usually defined as a (DP) < 10, are often interchangeably termed oligofructoses, inulo-oligosaccharides and fructo-oligosaccharides, represented by FOS.

Unless specified otherwise, the term inulin herein is meant to comprise both inulin and oligofructose.

Oligofructose, which is thus an inulin of which the composing polyfructose chains have a (DP) < 10 , is usually obtained by partial, acidic or enzymatic hydrolysis of inulin, but can also be obtained by enzymatic synthesis from sucrose, according to techniques well-known in the art.

Several grades of oligofructose are commercially available, for example from ORAFTI (Tienen, Belgium) under the brand name RAFTILOSE®, for example, RAFTILOSE® P95 which contains about 95 % by weight oligofructose with a degree of polymerisation (DP) ranging from 2 to 7 and contains about 5 % by weight in total of glucose, fructose and sucrose.

Due to the presence of β(2-1) fructosyl-fructose linkages between the fructosyl units of the inulin-type polyfructose chains, inulin is not digested by alimentary enzymes in the upper part of the digestive tract of humans and non ruminating mammals, but passes essentially unaltered into the lower part of the digestive tract to reach the colon where it is fermented by intestinal bacteria, while selectively promoting the growth of certain beneficial bacteria, particularly *Bifidobacteria* (Gibson et al., 1995). This bifidogenic effect, also called prebiotic effect, is known to exert beneficial effects on various physiological functions in the body.

In view of their almost non-digestibility by alimentary enzymes of humans and non ruminating mammals, fructans, levan-type fructans as well as inulin-type fructans, are generally considered as soluble dietary fibres.

In patent application EP 0 879 600, the use has been described of fructan-type carbohydrates, particularly chicory inulin, with an average degree of polymerisation ($\overline{DP}$) of at least 15, for the manufacture of a composition for the prevention and treatment of colon cancer in non-bovine mammals, particularly in humans. Optionally, said composition can also comprise a physiologically active substance, a drug or pro-drug, but no particulars have been disclosed in this respect

Furthennore, intraperitoneal injection of gamma-inulin, a specific polymorphic form of dahlia inulin, has been demonstrated to prolong the survival time of melanoma bearing mice (Cooper, 1986).

More recent studies revealed that non-digestible carbohydrates, including oligofructose and inulin, present inhibitory effects on the growth of intramuscularly transplanted mouse tumors (Taper et al., 1997).

Patent application EP 0 692 252 A1, discloses a composition containing inulin or oligofructose which presents a preventive effect on carcinogenesis and an inhibitory effect on the growth of cancer in mammals, particularly mammary cancer. EP 0 692 252 A1 also discloses compositions which in addition to inulin or oligofructose comprise conventional chemotherapeutic products actively destroying malignant tumor cells. In this way, the combination of the physiologically beneficial effects of non-digestible carbohydrates and of the curative effects of anti-cancer drugs are sought Furthermore, in Example 7 (page 12 of EP 0 692 252 A1) is mentioned that to determine potential synergistic therapeutic effects, a pharmaceutical composition comprising RAFTILINE® (i.e. chicory inulin) and a conventional chemotherapeutic product actively destroying malignant tumor cells, is prepared and a test is described wherein doxorubicine (an anti-cancer drug of the class of the antimitotic antibiotics) was injected to mice fed oligofructose/RAFTILINE® which were previously inoculated with L1210 leukaemic tumor cells. However, EP 0 692 252 A1 is completely silent about the outcome of the test and about possible synergistic effects between a non-digestible fructan-type carbohydrate and conventional anti-cancer drugs.

The combined effect of levan and four cytotoxic agents on the growth of experimental tumors in mice has been published (Leibovici et al., 1983). Additive effects were observed with all the combinations of levan and the cytotoxic agents, except for the combination of levan and methotrexate, an anti-metabolite anti-cancer agent, which gave a synergistic effect on Lewis lung carcinoma. However, no synergistic effect was observed for the combination of levan and 5-fluorouracil, an other anti-metabolite anti-cancer drug.

The problem

In view of the tremendous social and economical impact of cancer on individuals as well as on society in general, huge efforts are continuously made world-wide to find new or improved products, compositions and methods for the treatment of cancer. In particular, the search is continued in order to find highly effective drugs, preferably with a low degree of toxicity to normal cells, and compositions thereof, which are providing good therapeutic effects while provoking minimal discomfort to the patient.

Description of the invention

The present invention provides a solution to one or more of the problems mentioned above, by providing (i) a novel pharmaceutical composition comprising a synergistic combination of a non-digestible carbohydrate, i.e. an inulin (including inulin, oligofructose and mixtures thereof) and an anti-cancer drug, (III) the use of said combination for the manufacture of a medicament for the treatment of cancer in humans and in non-ruminating mammals.

By the term cancer is meant herein any kind of cancer occurring in humans and in non-ruminating mammals, irrespective of the stage of development of said cancer, thus including, in particular, the initiation, promotion, progression stages and the metastasis stage.

The invention is based on the findings made by the inventors that the combination of inulin and an anti-metabolic anti-cancer drug, not only results in an additive therapeutical effect vis-à-vis the carcinogenesis and the growth of cancer in humans and in non-ruminating mammals, but surprisingly provokes a synergistic therapeutical effect vis-à-vis said disease.

So, in one aspect the present invention relates to a novel pharmaceutical composition comprising a synergistic combination of inulin and an anti-metabolic anti-cancer drug , which combination provokes a synergistic therapeutical effect on the carcinogenesis and growth of cancer in humans and in non-ruminating mammals.

Typically suitable inulins include chicory inulin, in particular, inulin with a $(\overline{DP})$ of about 10, such as for example RAFTILINE® ST and RAFTILINE® LS, and inulin with a $(\overline{DP}) \geq 23$, such as, for example, RAFTILINE® HP and a suitable oligofructose is, for example, RAFTILOSE® P95, all from ORAFTI (Belgium).

The term therapeutical effect used herein refers to an inhibitory effect on the incidence and growth of cancer as well as to a curative effect provoking the decrease of the volume of an existing cancer and possibly the regression or curing of the cancer disease.

By anti-metabolic anti-cancer drug is meant a drug selected from said class of anti-cancer drugs which embraces products which compete with the normal metabolites of nucleic acids in cell synthesis and in cell growth pathways. This class of drugs is comprising methotrexate, cytarabin, fluorouracil, mercaptopurin, thioguanine, azathioprin and hydroxycarbamide.

It is understood that the term drug used herein includes the drug *per se* as well as a pro-drug, i.e. a compound which in the body is transformed into the corresponding drug.

The composition according to the invention is suitable in the first place for the treatment of cancer in humans. However, it is very suitable too for the treatment of cancer in non-ruminant mammals, such as for example horses, rabbits, dogs and cats.

In a preferred embodiment the inulin is a chicory inulin with a $(\overline{DP})$ of about 10. In another preferred embodiment the inulin is a chicory inulin with a $(\overline{DP})$ of $\geq 23$, typically of about 25 to about 30. In still another preferred embodiment, the inulin is an oligofructose, more preferably an oligofructose with a (DP) of 2 to 7.

In a further preferred embodiment, the anti-metabolic drug is 5-fluorouracil or methotrexate.

In a particularly preferred embodiment the inulin is chicory inulin or oligofructose or a mixture thereof and the anti-cancer drug is 5-fluorouracil or methotrexate.

Furthermore, a composition according to the present invention may comprise, instead of one anti-metabolic drug, a mixture of two or more anti-metabolic drugs as well as a mixture of one or more anti-metabolic drugs with one or more anti-cancer drugs belonging to a different class.

The composition according to the invention can be prepared by conventional methods, comprising, for example, mixing the desired amounts of the active components, optionally in combination with one or more pharmaceutically acceptable solvents, diluents, excipients, and/or additives commonly used in galenic pharmacy.

The compositions can be presented in conventional, well known galenic forms for oral, parenteral or rectal administration, or for tube feeding. The compositions for oral administration can for example be liquids, gels or solids and exist, for example, as tablets, coated tablets, coated pills, capsules, granules, solutions, syrups, suspensions or emulsions. The compositions for administration via tube feeding can for example be solutions, syrups, suspensions or emulsions which optionally can be mixed with normal food compositions for tube feeding. The compositions for rectal administration may for example be solids, gels or liquids, presented for example in the form of suppositories, gels, solutions, suspensions or emulsions. The composition for parenteral administration can for example be in the form of a solution, emulsion or suspension, suitable for said administration, including for subcutaneous, intramuscular, intraveneous and intraperitoneal administration.

Suitable galenic forms also include retard release forms as well as sustained release forms which are well known in the art.

The composition according to the invention comprises at least an effective dose of the inulin as well as of the anti-cancer drug or drug mixture. A dose which is effective for treatment of cancer can be formulated in the form of a single dose unit or of several partial dose units, the administration of which can be spread over a certain period of time. Usually the composition according to the invention will be administered spread over several partial dose units, and spread, possibly with certain intervals, over one or more days or weeks.

The effective dose of the synergistic combination of inulin and anti-metabolic anti-cancer drug may depend on various factors, including the affected being, a human or species of non-ruminating mammals, its age and physical condition, the kind and stage of development of the cancer, the kind of inulin and of anti-cancer drug, and the method of administration of the synergistic combination. The effective dose, the optimal galenic form and unit dose, and the way of administration can be determined by the skilled person following conventional methods.

A particularly interesting feature of the compositions according to the invention resides in the fact that the active ingredients of the synergistic combination, i.e. the inulin and the anti-metabolic anti-cancer drug, can be administered to the human or non ruminating mammal (i) simultaneously and present in the same galenical formulation (constituting the pharmaceutical composition according to the present invention) , or (ii) simultaneously but present in two separate galenic formulations (constituting together the pharmaceutical composition according to the present invention) and optionally via two different methods of administration, as well as (iii) separately, i.e. non simultaneously, via two separate galenic formulations (constituting together the pharmaceutical composition according to the present invention) and optionally via two different methods of administration.

It is understood that in the above mentioned methods of administration, the inulin component as well as the anti-cancer drug of the composition of the invention, can be administered orally, via tube feeding, parenterally or rectally.

Furthermore, the combined composition or each of the active components can be administered locally or systemically, and they, accordingly, have to be present in a galenic formulation which is suitable for the method of administration.

When the fructan component and the anti-cancer drug component of the combination according to the invention are not simultaneously administered to the human or mammal, it is of course compulsory that they are brought into the body of said being in such a manner that their functional effects on the human or mammal are simultaneously present in order to provoke the synergistic effect in accordance with the present invention.

According to one preferred embodiment, both active ingredients are administered simultaneously in one pharmaceutical composition to the human or mammal.

In an other preferred embodiment, both active ingredients are administered simultaneously but in separate formulations. Optionally a different way of administration for each of the formulations can be used.

In a further preferred embodiment both active ingredients are administered separately in separate formulations and optionally via different methods of administration and/or at different time periods.

If the active ingredients are not administered simultaneously via the same formulation, the anti-cancer drug is preferably administered orally or parenterally, whereas the inulin is preferably administered orally, via tube feeding or parenterally. The inulin can be administered in various galenic formulations, such as for example tablets, capsules, syrups, solutions, suspensions or emulsions. The inulin can even be administered orally in the form of a functional food or feed, i.e. a food or feed product which during its manufacture has been provided with the desired amount of inulin, such as, for example, dairy products e.g. yoghurts or cheeses; jams or marmalades; baked goods e.g. biscuits, breads or breakfast cereals; desserts e.g. puddings or ice-creams; table spreads, e.g. margarines; drinks; meal replacers; or confectionery, e.g. gums.

In a further preferred embodiment, inulin is already administered to the human or mammal some time, for example one week, before the composition of the invention is administered. Without wishing to be bound by any theory, it is supposed that as a result of the bifidogenic effect of the previously administered inulin, the intestinal flora and/or the effected functions have been brought into a condition in which the synergistic effect provoked by the combination of active ingredients according to the invention can develop optimally.

In a further aspect the invention relates to a pharmaceutical composition as defined herein above for the treatment of cancer in humans or in non-ruminating mammals.

In still a further aspect, the invention relates to the use of a combination of inulin and an anti-metabolic anti-cancer drug for the manufacture of a pharmaceutical composition as defined herein above for the treatment of cancer in humans or in non-ruminating mammals.

Examples

In support of the present invention, the following illustrative data are given regarding experiments wherein the therapeutical effects on cancer in mice of a composition according to the present invention are compared with the therapeutical effects of a composition containing a combination of inulin with an anti-cancer drug which belongs to a different class.

To investigate whether dietary treatment with inulin ( inulin or oligofructose [FOS] ) would be able to potentiate the therapeutic effects of anti-cancer drugs commonly utilised in human cancer treatment, representatives of 4 principal classes of anti-cancer drugs have been chosen and compared in the following test.

The drugs were: endoxan (belonging to the class of alkylating agents), adriblastina (adriamycin) ( belonging to the class of antimitotic antibiotics), 5-fluorouracil (belonging to the class of anti-metabolic agents) and oncovin (belonging to the class of antineoplastic alkaloids).

Viable, neoplastic cells ($10^6$) from the ascitic form of a transplantable mouse liver tumor (TLT) (Taper, 1966 and Cappucino, 1966) were intraperitoneally transplanted into 12 per group young adult NMRI male mice weighing about 30 g, supplied by "Animalerie Facultaire", UCL, Brussels. Mice of the experimental groups were fed with a basal diet supplemented with 15 % FOS or inulin starting 7 days before tumor transplantation up to the end of the experiment. Control animals received the basal diet for experimental animals AO4 furnished by UAR, Villemoisson sur Orge, France, and water ad libitum.

A single, subtherapeutic dose of cytotoxic drugs was intraperitoneally injected 48 hours after intraperitoneal TLT tumor transplantation at following doses: endoxan 80 mg/kg; adriamycin 0.1 mg/kg; oncovin 0.5 mg/kg and 5-fluorouracil 40 mg/kg.

The criteria for the evaluation of results were the mortality rates expressed by mean survival time (MST) and the percentage of increase of life span (ILS) in experimental groups compared with the controls, calculated according to the NCI instructions (Geron et al., 1972). The results of each experiment were confirmed by another experiment performed at another time and were cumulatively calculated (as presented in Table 1). The results concerning the survival rates of mice bearing the ascitic form of a TLT tumor, observed in 2 separately performed experiments on each type of anti-cancer drug (12 mice per group), are cumulatively presented in Table 1 below.

In all the experiments a chemotherapy potentiating effect has been observed for dietary FOS or inulin. Obviously this therapeutic effect was somewhat different for the various drugs. Only for 5-fluorouracil treatment a synergistic therapeutic effect has bee observed. For treatment with oncovin, endoxan and adriamycin, the therapeutic effects had only additive character. There is no significant difference observed between treatment with inulin or FOS.

All results of supplementary dietary FOS treatment were statistically very highly significant (p < 0.001). There were no negative results in any of the separately performed experiments. 'Furthermore, no gastro-intestinal troubles were observed in all animals after the direct introduction of 15 % FOS or inulin in the diet. There was no sign of increased drug toxicity after dietary introduction of both inulin or FOS (e.g. there was no difference in body weights between mice treated only with a cytotoxic drug and those treated with supplementary inulin or FOS).

Furthermore, it should be noted that in the experiments unfavourably therapeutic conditions were deliberately utilised by the administration of a single, sub-therapeutic dose of cytotoxic drug 48 hours after the intraperitoneal transplantation of a very aggressive and rapidly growing ascitic form of mouse tumor.

In a further experiment, carried out in a similar manner as the previous experiment, the effects were examined of a composition according to the invention, containing a combination of the anti-metabolite anti-cancer drug methotrexate and inulin or FOS, in comparison with the effects of the single components.

The effects were evaluated via the survival criteria of N.M.RI mice bearing ascitic TLT tumors, treated with a single i.p. dose of 20 mg/kg methotrexate (10 investigated mice in each group) and the results are presented in Table 2 below.

TABLE 1.

| Therapeutic effects of FOS or inulin associated to a single dose of different cytotoxic drugs administered to ascitic TLT tumor bearing mice | | | |
|---|---|---|---|
| Treatment | ILS (%) | Effect of combined treatment | Statistical significance |
| 5-Fluorouracil * | 18.75 | | |
| FOS * | 12.5 | | |
| 5-Fluorouracil + FOS | 40.6 | Synergistic | p < 0.001 |
| Adriamycin * | 14.7 | | |
| FOS* | 5.9 | | |
| Adriamycin + FOS * | 17.6 | Additive | p < 0.001 |
| Endoxan * | 0 | | |
| FOS * | 25.0 | | |
| Endoxan + FOS * | 25.0 | Additive | p < 0.001 |
| Oncovin * | 33.33 | | |
| FOS * | 13.33 | | |
| Inulin * | 10.0 | | |
| Oncovin + FOS * | 46.66 | Additive | p < 0.001 |
| Oncovin + Inulin * | 43.33 | Additive | p < 0.01 |

*Each of the differently treated group of 12 mice had its individual, untreated, control group to which it was compared in the calculation of the increase of life span (ILS). The cumulatively presented results are based each on 2 experiments independently performed at different time. Lagrank test was utilised for statistical analysis of the results.*

*: comparative test

TABLE 2.

| Therapeutic effects of FOS or inulin associated to a single dose of methotrexate administered i.p. at 20 mg/kg to ascitic TLT tumor bearing mice | | |
|---|---|---|
| **Treatment** | **ILS (%)** | **Effect of combined treatment** |
| untreated (control) * | | |
| methotrexate * | 2 | |
| FOS* | 5 | |
| inulin * | 11 | |
| methotrexate + FOS | 29 | Synergistic |
| methotrexate + inulin | 20.5 | Synergistic |

*: comparative test

From the comparative data presented in Tables 1 and 2, it clearly follows that the composition according to the present invention comprising a combination of inulin and an anti-metabolic anti-cancer drug has a synergistic therapeutical effect on cancer in mice, whereas for the compositions containing inulin and an anti-cancer drug of an other class only an additive effect is observed. Besides, the compositions of the invention provoked no disadvantageous effects on the digestive tract and were not potentiating the toxicity of the anti-cancer drug.

As a result of said synergistic effect the compositions of the invention present considerable advantages over conventional anti-cancer compositions. For example, compared to a conventional composition containing the same concentration of anti-cancer drug, the therapeutical effect of a composition of the invention is significantly increased which may lead to a considerable improvement of the bodily condition of the treated human or mammal and which may enable to better control the disease and even enable to have restored to a more or lesser extent the normal physiological functions and affected body structures. On the other hand, said synergistic effect can enable the use of certain anti-cancer drugs, which are fairly toxic or provoke disadvantageous side effects, in compositions according to the invention in reduced concentrations while still maintaining the same or a desirable level of therapeutical effect. Said synergistic effect makes it even possible to use certain anti-cancer drugs which, at the concentration they have to be used in conventional composition to obtain a desired therapeutical effect, are too toxic or provoke such undesirable side effects that they can not be supported by the treated human or mammal.

In addition, the treated human or mammal also enjoys the known beneficial effects resulting from the bifidogenic effect of the inulin which is present in the composition of the invention.

Consequently, it can be concluded that the composition according to the invention present a considerable improvement in the fight against cancer in humans and in non-ruminating mammals.

REFERENCES

Williams, C.M., Dickerson, J.W., Nutr. Res. Rev., 3, 45-100, (1990)
Roberfroid, M.B., Mutation Res., 259, 351-362, (1991)
Milner, J.A., Functional Foods, pp. 39-70, Ed. Van Nostrand Reinhold Publishing Company, New York, (1994)
Roberfroid, M.B., World of ingredients, 3, 42-44 (1995)
Nossal, G.J.V., Sci. Amer., 269, 21-30 (1993)
Perdigon, G. et al., Int. J. Immunother., 9, 29-52 (1993)
Wattenberg, L., Cancer; Res., 52, 2085s-2091s, (1992)
Potter, J. et al., Principles of Chemoprevention, IARC Scientific Publication N° 139, 61-90, (1996);
Howe, G.R. et al., J. Natl. Cancer Inst., 84, 1887-1896, (1992);
Reddy, B.S. et al., Gastroenterol., 102, 1475-1482, (1992).
De Leenheer, L., Carbohydrates as Organic Raw Materials, Vol. III, p.67-92, (1996)
Gibson, G.R. et al., Gastroenterology,108, 975-982, (1995) and J. Nutr., 125, 1401-1412, (1995)
Cooper, P.D. et al., Mol.Immunol., 23, 903-908, (1986)
Taper, H.S. et al., Int. J. Cancer, 71, 1109-1112 (1997).
Taper, H.S. et al., Cancer Res., 26, 193-198, (1966)
Cappucino, J.G. et al., Cancer Res., 26, 689-694, (1966)
Geron; R.J. et al., Cancer Chemother. Reports, 3, 1-103, (1972).

**Claims**

1. Pharmaceutical composition **characterised in that** it comprises a combination of an effective dose of inulin and of an anti-metabolic anti-cancer drug.

2. Pharmaceutical composition according to claim 1 wherein the inulin is inulin with a DP up to about 100, or oligofructose or a mixture thereof.

3. Pharmaceutical composition according to claim 1 or claim 2, wherein the inulin is chicory inulin with a $(\overline{DP})$ ranging from about 10 to about 30, or oligofructose with a DP ranging from 2 to 7 and containing about 5 wt% in total of glucose, fructose and sucrose.

4. Pharmaceutical composition according to any one of claims 1 to 3 wherein the anti-cancer drug is selected from the group consisting of methotrexate, cytarabin, fluorouracil, mercaptopurin, thioguanin, azathioprin and hydroxycarbamide.

5. Pharmaceutical composition according to claim 4 wherein the anti-cancer drug is 5-fluorouracil or methotrexate.

6. Pharmaceutical composition according to any one of claims 1 to 5 which additionally to the said anti-metabolic anti-cancer drug contains one or more anti-cancer drugs belonging to the class of anti-metabolic anti-cancer drugs and/or to another class of anti-cancer drugs.

7. Pharmaceutical composition according to any one of claims 1 to 6 in which the inulin and the anti-metabolic anti-cancer drug which constitute the combination are simultaneously present in the same galenic formulation.

8. Pharmaceutical composition according to any one of claims 1 to 6 in which the inulin and the anti-metabolic anti-cancer drug which constitute the combination are present in separate formulations which together form the pharmaceutical composition.

9. Pharmaceutical composition according to any one of claims 1 to 8 wherein the single galenic formulation or the separate galenic formulations forming the pharmaceutical composition are suitable for oral, parenteral or rectal administration, or for tube feeding.

10. Pharmaceutical composition according to claim 8 in which the inluin is present in a functional food or feed.

11. Pharmaceutical composition according to claim 8 in which the anti-cancer drug is present in a formulation which is suitable for oral or parenteral administration.

12. Pharmaceutical composition according to any one of claims 1 to 11 for use as a medicament for the treatment of cancer in humans.

13. Pharmaceutical composition according to any one of claims 1 to 11 for use as a medicament for the treatment of cancer in non-ruminating mammals.

14. Use of a combination of inulin and an anti-metabolic anti-cancer drug for the manufacture of a pharmaceutical composition as defined in any one of claims 1 to 11 for the treatment of cancer in humans or in a non-ruminating mammal.

**Patentansprüche**

1. Pharmazeutische Zusammensetzung, **dadurch gekennzeichnet, dass** sie eine Kombination einer wirksamen Dosis Inulin und einer antimetabolischen, krebsbekämpfenden Substanz umfasst.

2. Pharmazeutische Zusammensetzung nach Anspruch 1, wobei das Inulin ein Inulin mit einem DP bis zu ca. 100 oder Oligofructose oder eine Mischung davon ist.

3. Pharmazeutische Zusammensetzung nach Anspruch 1 oder Anspruch 2, wobei das Inulin ein Chicoréeinulin mit

einem ($\overline{DP}$) im Bereich von ca. 10 bis 30 oder Oligofructose mit einem DP im Bereich von 2 bis 7 ist und ca. 5 Gew.-% Glucose, Fructose und Sucrose insgesamt enthält.

4.  Pharmazeutische Zusammensetzung nach einem der Ansprüche 1 bis 3, wobei die krebsbekämpfende Substanz aus der Gruppe bestehend aus Methotrexat, Cytarabin, Fluorouracil, Mercaptopurin, Thioguanin, Azathioprin, Hydroxycarbamid ausgewählt ist.

5.  Pharmazeutische Zusammensetzung nach Anspruch 4, wobei die krebsbekämpfende Substanz 5-Fluorouracil oder Methotrexat ist.

6.  Pharmazeutische Zusammensetzung nach einem der Ansprüche 1 bis 5, die zusätzlich zur antimetabolischen, krebsbekämpfenden Substanz eine oder mehrere krebsbekämpfende Substanzen, die der Klasse der antimetabolischen, krebsbekämpfenden Substanzen und/oder einer anderen Klasse von krebsbekämpfenden Substanzen angehören, enthält.

7.  Pharmazeutische Zusammensetzung nach einem der Ansprüche 1 bis 6, in der das Inulin und die antimetabolische, krebsbekämpfende Substanz, welche die Kombination bilden, in derselben galenischen Formulierung gleichzeitig enthalten sind.

8.  Pharmazeutische Zusammensetzung nach einem der Ansprüche 1 bis 6, in der das Inulin und die antimetabolische, krebsbekämpfende Substanz, welche die Kombination bilden, in gesonderten Formulierungen vorhanden sind, die gemeinsam die pharmazeutische Zusammensetzung bilden.

9.  Pharmazeutische Zusammensetzung nach einem der Ansprüche 1 bis 8, wobei die einzelne galenische Formulierung oder die gesonderten galenischen Formulierungen, welche die pharmazeutische Zusammensetzung bilden für eine orale, parenterale oder rektale Verabreichung oder für die Sondenernährung geeignet sind.

10. Pharmazeutische Zusammensetzung nach Anspruch 8, in der das Inulin in einem Nahrungs- oder Futtermittel enthalten ist.

11. Pharmazeutische Zusammensetzung nach Anspruch 8, in welcher die krebsbekämpfende Substanz in einer Formulierung vorhanden ist, die für die orale oder parenterale Verabreichung geeignet ist.

12. Pharmazeutische Zusammensetzung nach einem der Ansprüche 1 bis 11 zur Anwendung als Medikament für die Behandlung von Krebs bei Menschen.

13. Pharmazeutische Zusammensetzung nach einem der Ansprüche 1 bis 11 zur Anwendung als Medikament für die Behandlung von Krebs bei nicht wiederkäuenden Säugetieren.

14. Anwendung einer Kombination von Inulin und einer antimetabolischen, krebsbekämpfenden Substanz zur Herstellung einer pharmazeutischen Zusammensetzung, wie in einem der Ansprüche von 1 bis 11 definiert, zur Behandlung von Krebs bei Menschen oder nicht wiederkäuenden Säugetieren.

**Revendications**

1.  Composition pharmaceutique, **caractérisée en ce qu'**elle comprend une combinaison d'une dose efficace d'inuline et d'un médicament anticancéreux antimétabolique.

2.  Composition pharmaceutique suivant la revendication 1, dans laquelle l'inuline est une inuline ayant un (DP) allant jusqu'à environ 100, ou un oligofructose ou un mélange de ceux-ci.

3.  Composition pharmaceutique suivant les revendications 1 ou 2, dans laquelle l'inuline est une inuline de chicorée ayant un (DP) moyen compris dans la gamme d'environ 10 à environ 30, ou un oligofructose ayant un (DP) compris dans la gamme de 2 à 7 et contenant environ 5% en poids de glucose, de fructose et de sucrose.

4.  Composition pharmaceutique suivant l'une quelconque des revendications 1 à 3, dans laquelle le médicament anticancéreux est choisi dans le groupe consistant en méthotrexate, cytarabine, fluoro-uracile, mercaptopurine, thioguanine, azathioprine et hydroxycarbamide.

5. Composition pharmaceutique suivant la revendication 4, dans laquelle le médicament anticancéreux est le 5-fluoro-uracile ou le méthotrexate.

6. Composition pharmaceutique suivant l'une quelconque des revendications 1 à 5, qui en plus dudit médicament anticancéreux antimétabolique contient un ou plusieurs médicaments anticancéreux appartenant à la classe des médicaments anticancéreux antimétaboliques et/ou à une autre classe de médicaments anticancéreux.

7. Composition pharmaceutique suivant l'une quelconque des revendications 1 à 6, dans laquelle l'inuline et le médicament anticancéreux antimétabolique qui constituent la combinaison sont présents simultanément dans la même formulation galénique.

8. Composition pharmaceutique suivant l'une quelconque des revendications 1 à 6, dans laquelle l'inuline et le médicament anticancéreux antimétabolique qui constituent la combinaison sont présents dans des formulations séparées qui forment ensemble la composition pharmaceutique.

9. Composition pharmaceutique suivant l'une quelconque des revendications 1 à 8, dans laquelle la formulation galénique unique ou les formulations galéniques séparées formant la composition pharmaceutique conviennent pour l'administration orale, parentérale ou rectale, ou pour une alimentation par sonde.

10. Composition pharmaceutique suivant la revendication 8, dans laquelle l'inuline est présente dans l'alimentation ou les aliments fonctionnels.

11. Composition pharmaceutique suivant la revendication 8, dans laquelle le médicament anticancéreux est présent dans une formulation qui convient pour l'administration orale ou parentérale.

12. Composition pharmaceutique suivant l'une quelconque des revendications 1 à 11, utile en tant que médicament pour le traitement du cancer chez les humains.

13. Composition pharmaceutique suivant l'une quelconque des revendications 1 à 11, utile en tant que médicament pour le traitement du cancer chez des mammifères non ruminants.

14. Utilisation d'une combinaison d'inuline et d'un médicament anticancéreux antimétabolique pour la fabrication d'une composition pharmaceutique suivant l'une quelconque des revendications 1 à 11, pour le traitement du cancer chez les humains ou chez un mammifère non ruminant.